# FASCICULE DE BREVET EUROPEEN

(11) **EP 1 326 661 B1**
(45) Date de publication et mention de la délivrance du brevet: **15.12.2004**
(21) Numéro de dépôt: 01978569.0
(22) Date de dépôt: 18.10.2001
(51) Int. Cl.: A61M 5/20

(54) **SERINGUE D'AUTO-INJECTION D'UN MELANGE EXTEMPORANE**
SPRITZE ZUR SELBSTINJEKTION FÜR UNMITTELBARE VERMISCHUNG VON MEDIKAMENTEN
SYRINGE FOR AUTOMATIC INJECTION OF AN EXTEMPORANEOUS MIXTURE

(30) Priorité: 19.10.2000 FR 0013405
(43) Date de publication de la demande: 16.07.2003
(73) Titulaire: SEDAT, 69540 Irigny (Rhône) (FR)
(72) Inventeur: ARNISSOLLE, Yves, F-69230 Saint-Genis-Laval (FR)
(74) Mandataire: Blot, Philippe Robert Emile
(86) Numéro de dépôt international: PCT/FR2001/003232
(87) Numéro de publication internationale: WO 2002/034316

(56) Documents cités:
- EP-A- 0 405 320
- EP-A- 0 562 671
- WO-A-94/09839

## Description

La présente invention concerne une seringue d'injection d'un mélange extemporané, du type comportant :
- un corps renfermant au moins deux substances initialement séparées par un piston intermédiaire, lequel corps comporte deux parties mobiles l'une par rapport à l'autre, entre une position initiale dans laquelle les deux substances sont séparées et une position finale dans laquelle les deux substances sont mélangées pour former un mélange extemporané ;
- des moyens automatiques d'injection du mélange extemporané hors du corps afin de procéder à l'injection ; et
- des moyens de blocage des moyens automatiques d'injection interdisant leur déclenchement.

Une telle seringue est notamment décrite dans le document EP-A-0.219.899 en regard des figures 14 et 15 ou le document WO 94/09839.

Dans ce document, la seringue comporte une cartouche d'injection reçue dans un boîtier de seringue. Cette cartouche constitue un réservoir tubulaire dans lequel sont retenues les substances à injecter. Ces dernières sont séparées initialement par des pistons mobiles. Les moyens de mélange des substances sont formés par un bossage latéral de la cartouche. Celui-ci augmente localement la section interne de la cartouche et délimite un passage permettant aux substances de contourner les pistons séparateurs.

Des moyens d'actionnement automatique de la paroi arrière en vue de l'injection du mélange extemporané sont en outre prévus dans le boîtier. Ils comportent essentiellement un poussoir chargé par un ressort et des moyens de libération du poussoir. Afin d'éviter un déclenchement accidentel des moyens d'injection du mélange extemporané, il est prévu un organe amovible de blocage des moyens automatiques d'injection. Cet organe de blocage comporte une broche initialement engagée entre des languettes élastiques retenant le ressort. Cette broche interdit la libération du ressort en interdisant la déformation des languettes.

Afin d'assurer la reconstitution du mélange extemporané, au moins une partie du boîtier de seringue est déplaçable par rapport à la cartouche, de telle sorte que, lorsque cette partie du boîtier est amenée manuellement à coulisser sur la cartouche, le poussoir sollicite la cloison arrière mobile de la cartouche et déplace celle-ci vers la cloison avant conduisant ainsi au mélange des substances à injecter par passage de celles-ci au travers du bossage latéral.

Après avoir procédé au mélange des substances à injecter et après retrait de l'organe de blocage, le ressort des moyens d'actionnement est libéré, ce qui provoque l'injection automatique des substances préalablement mélangées.

Dans la pratique, on constate que l'opérateur libère parfois le ressort des moyens d'actionnement pour procéder à l'injection avant que le mélange des substances ne soit achevé, la partie mobile du boîtier n'ayant pas été amenée à coulisser suivant toute sa course le long de la cartouche. Le mélange extemporané n'est alors pas réalisé ou n'est réalisé que de manière incomplète, de sorte que sa concentration en les différents constituants contenus initialement dans la cartouche est mauvaise.

L'effet de ce dysfonctionnement est d'autant plus sensible que les seringues d'auto-injection sont couramment utilisées dans des situations de crise où une personne seule est amenée à procéder à une injection sur elle-même. Le stress conduit alors l'utilisateur à agir précipitamment sans prendre garde à la reconstitution effective et complète du mélange.

L'invention a pour but de proposer une solution à ce problème, évitant ainsi que le mélange extemporané ne soit réalisé que de manière incomplète lors de l'injection.

A cet effet, l'invention a pour objet une seringue du type précité, caractérisée en ce qu'elle comporte des moyens de verrouillage des moyens de blocage interdisant le déblocage des moyens automatiques d'injection avant la fin du mélange des deux substances.

Suivant des modes particuliers de réalisation, la seringue comporte l'une ou plusieurs des caractéristiques suivantes :
- les moyens de blocage comportent un organe de blocage déplaçable entre une position active de blocage des moyens automatiques d'injection, et une position inactive de blocage des moyens automatiques d'injection, les moyens de verrouillage comportant des profils complémentaires d'accrochage par enclenchement élastique prévus sur ledit organe de blocage et sur une première partie mobile du corps, lesquels profils complémentaires d'accrochage sont normalement en prise, et la seconde partie mobile du corps comporte au moins une surface de dégagement des profils complémentaires d'accrochage adaptés pour assurer le dégagement desdits profils complémentaires d'accrochage, seulement lorsque les deux parties mobiles du corps sont dans leur position finale ;
- lesdits profils complémentaires d'accrochage comportent, d'une part, au moins un crochet solidaire dudit organe de blocage, et, d'autre part, au moins une lumière ménagée dans ladite première partie mobile, le ou chaque crochet étant sollicité élastiquement pour être reçu au moins partiellement dans une lumière associée, et la ou chaque surface de dégagement est adaptée pour s'étendre dans la ou chaque lumière et repousser totalement le ou chaque crochet associé hors de la lumière, seulement lorsque les deux parties mobiles sont dans leur position finale ; et
- ledit organe de blocage (73) est amovible.

L'invention sera mieux comprise à la lecture de la description qui va suivre, donnée uniquement à titre d'exemple et faite en se référant aux dessins sur lesquels :
- la figure 1A est une vue en coupe longitudinale d'une seringue selon l'invention, avant utilisation ;
- la figure 1 B est une vue à plus grande échelle en coupe de l'extrémité arrière de la seringue de la figure 1A ;
- la figure 2 est une vue en perspective du manchon intermédiaire de la seringue selon l'invention ;
- la figure 3 est une vue en perspective du couvercle arrière de la seringue ;
- la figure 4 est une vue en perspective de la poignée de préhension de la seringue ;
- la figure 5 est une vue en perspective de l'organe de blocage de la seringue ;
- la figure 6A est une vue en coupe longitudinale de la seringue à l'issue de la phase de reconstitution du mélange extemporané ;
- la figure 6B est une vue en coupe longitudinale à plus grande échelle de l'extrémité arrière de la seringue dans l'état de la figure 6A ;
- la figure 7A est une vue en coupe longitudinale de la seringue, avant injection, l'organe de blocage étant retiré ;
- la figure 7B est une vue en coupe longitudinale à plus grande échelle de l'extrémité arrière de la seringue dans l'état de la figure 7A ; et
- la figure 8 est une vue en coupe longitudinale de la seringue, après injection.

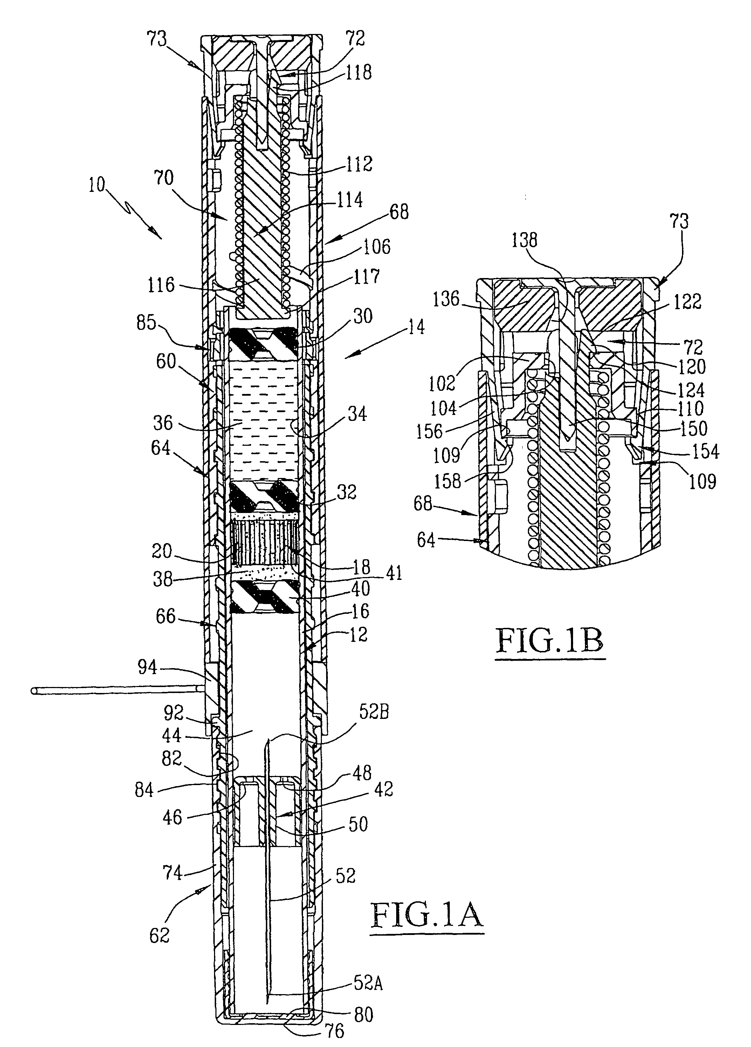

Une seringue d'auto-injection 10 selon l'invention est représentée sur la figure 1 dans son état initial correspondant à son état de stockage avant utilisation. Elle comporte une cartouche d'injection 12 et un boîtier extérieur 14 dans lequel la cartouche est montée.

La cartouche d'injection 12 comporte un tube sensiblement cylindrique 16. La surface intérieure du tube est généralement cylindrique de section circulaire. Dans sa partie intermédiaire, le tube comporte un tronçon médian 18 où sa surface intérieure présente un profil délimitant au moins un passage d'écoulement permettant le mélange extemporané de deux substances initialement séparées contenues dans le tube.

Le tronçon médian 18 est bordé de part et d'autre par des tronçons à surface intérieure cylindrique lisse dépourvue de profils en saillie ou en creux.

Le tronçon 18 présente un ensemble de nervures 20 s'étendant parallèlement les unes aux autres suivant des génératrices du tube. Ces nervures 20 font saillie par rapport à la surface cylindrique intérieure du tube. Elles définissent entre elles des canaux parallèles formant des passages pour l'écoulement d'un fluide.

Depuis une extrémité arrière, la cartouche d'injection 12 comporte une cloison mobile 30 formant piston dont la surface latérale cylindrique est munie de nervures périphériques afin d'assurer l'étanchéité aux liquides et aux gaz entre celle-ci et la surface latérale intérieure du tube 16.

Un piston intermédiaire 32 est disposé plus en avant dans le tube mais en arrière du tronçon 18. Il délimite avec la cloison 30 un logement 34 pour un solvant 36. Le piston intermédiaire 32 présente sur sa surface latérale des nervures périphériques afin d'assurer l'étanchéité aux liquides et aux gaz avec la surface intérieure du tube 16. Ce piston présente une longueur inférieure à celle des nervures 20.

A l'avant du piston intermédiaire 32, au niveau du tronçon 18, est disposé un amas de poudre 38 contenant une substance médicalement active. Il est maintenu contre le piston intermédiaire 32 par une cloison mobile avant 40 identique à la cloison mobile arrière 30. Le piston 32 et la cloison 40 délimitent ainsi initialement une chambre 41 de confinement de la poudre 38.

L'amas de poudre 38 peut être remplacé par une substance liquide.

Un porte-aiguille coulissant 42 formant une paroi avant de la cartouche est disposé au-delà de la cloison 40 vers l'avant du tube 16. Il est représenté sur la figure 1 en position escamotée dans le tube.

Le porte-aiguille 42 et la cloison avant 40 délimitent un espace libre 44 empli d'air. Le porte-aiguille 42 est formé par une cuvette 46 en matière plastique dont le fond est dirigé vers la cloison avant 40 et dont la paroi latérale est appliquée à coulissement contre la surface interne du tube 16. La cuvette 46 comporte des évents 48 permettant la fuite vers l'extérieur de l'air contenu dans l'espace 44. La cuvette présente, venu de matière avec son fond, un plot axial intérieur 50 de fixation d'une aiguille d'injection 52.

L'aiguille 52 dépasse de part et d'autre de la cuvette 46. Elle est initialement totalement reçue à l'intérieur du tube 16. Elle présente à ses extrémités une pointe biseautée avant 52A et une pointe biseautée arrière 52B. Cette dernière extrémité forme une extrémité de perforation de la cloison avant 40.

Le boîtier 14 comporte essentiellement un corps 60, formé d'un couvercle avant 62 et d'un couvercle arrière 64, reliés l'un à l'autre par un manchon intermédiaire 66, une poignée de préhension 68 mobile par rapport au corps 60, des moyens automatiques 70 d'actionnement de la cloison arrière mobile de la cartouche en vue de l'injection, et des moyens 72 de libération des moyens d'actionnement 70. Les moyens automatiques d'actionnement 70 associés aux moyens de libération 72 sont adaptés pour assurer, lors de leur déclenchement, l'injection d'un mélange extemporané préalablement préparé dans la seringue. Le boîtier comporte en outre un organe 73 de blocage des moyens automatiques 70 d'actionnement de la cloison arrière.

Le couvercle avant 62 est lié à demeure au manchon intermédiaire 66 par vissage et enclenchement élastique de profils complémentaires en saillie et en creux. Ils forment ensemble avec le tube 16 de la cartouche qui s'appuie sur le couvercle avant 62, un réservoir tubulaire de la seringue par rapport auquel le couvercle arrière 64 est mobile afin de déplacer la cloison arrière 30 suivant une course de préparation du mélange extemporané.

Le couvercle avant 62 comporte un tronçon tubulaire 74. Son diamètre intérieur est supérieur au diamètre extérieur du tube 16. Le couvercle 62 comporte une paroi obturante 76 au centre de laquelle est définie une région de moindre épaisseur perforable par l'aiguille d'injection 52. L'extrémité avant du tube 16 s'appuie axialement sur la paroi obturante 76. Entre celles-ci est interposée une membrane perforable 80 assurant initialement l'isolement de l'aiguille 52 par rapport au milieu extérieur.

Sur la surface intérieure du tronçon tubulaire 74, le couvercle avant présente un filetage intérieur 82 auquel est relié par vissage le manchon intermédiaire 66.

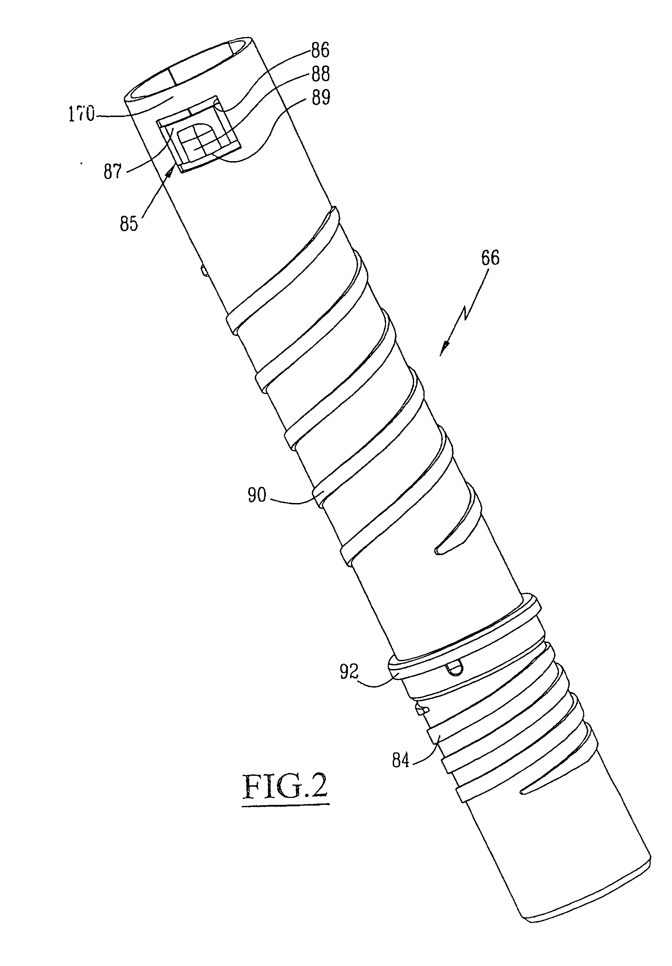

Ce manchon intermédiaire, représenté seul sur la figure 2, présente une forme générale tubulaire. Son diamètre intérieur est constant et correspond sensiblement au diamètre extérieur de la cartouche 12.

A son extrémité avant orientée vers l'aiguille d'injection 52, le manchon intermédiaire 66 présente un filetage extérieur 84 adapté pour coopérer avec le filetage 82, ménagé intérieurement dans le couvercle avant 62.

Au voisinage de son extrémité arrière, le manchon intermédiaire 66 comporte des organes élastiques 85 d'immobilisation du manchon intermédiaire 66 par rapport au couvercle arrière 64. Ces organes élastiques d'immobilisation 85 sont venus de matière avec le manchon intermédiaire 66. Ils sont disposés dans deux lumières débouchantes 86, diamétralement opposées, ménagées dans la partie courante du manchon intermédiaire 66 au voisinage de son extrémité arrière.

Chaque organe d'immobilisation 85 comporte une jambe élastique 87 reliée au manchon suivant un bord de la lumière 86, la liaison s'effectuant suivant une génératrice du manchon. En outre, chaque organe d'immobilisation comporte une saillie 88 ménagée sur la surface externe de la jambe élastique 87. Cette saillie définit, avec le bord de chaque jambe 87 tourné vers l'avant du manchon intermédiaire, une surface de butée 89 propre à coopérer avec une surface complémentaire du couvercle arrière afin d'assurer leur immobilisation axiale dans une position prédéterminée, la saillie 88 étant reçue dans un logement complémentaire du couvercle arrière, comme cela sera décrit ultérieurement.

Les jambes 87 sont naturellement sollicitées vers l'extérieur, les saillies 88 faisant alors saillies par rapport à la surface cylindrique externe du manchon intermédiaire 66.

Dans sa partie courante, entre les lumières 86 et le filetage 84, le manchon intermédiaire 66 présente un filetage 90 dont le sens des filets est le même que celui des filets du filetage 84.

Le pas du filetage 90 est grand. Ainsi, par exemple, le filet hélicoïdal formant le filetage 90 s'enroule sur trois tours sur la longueur du manchon 66.

Entre les deux filetages 84 et 90, le manchon intermédiaire 66 présente une butée périphérique 92 de blocage en translation d'une bague 94 de blocage de sécurité amovible, visible sur la figure 1A.

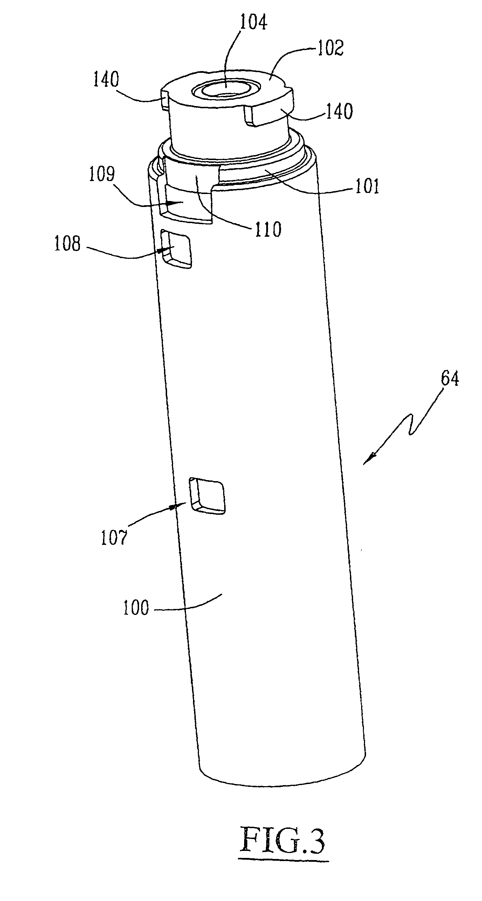

Le couvercle arrière 64, représenté seul sur la figure 3, est formé par un tronçon tubulaire 100 de section intérieure circulaire constante égale à la section extérieure du manchon intermédiaire 66. Vers l'arrière, le tronçon tubulaire 100 est prolongé par un tronçon cylindrique coaxial 101 présentant des diamètres intérieur et extérieur inférieurs à ceux du tronçon tubulaire 100. L'extrémité arrière du tronçon cylindrique 101 est partiellement obturée par un fond 102 présentant un passage central 104.

A son extrémité ouverte opposée au fond 102, le couvercle arrière 64 présente intérieurement un filetage 106 visible sur la figure 1A adapté pour coopérer avec le filetage 90 ménagé sur la surface extérieure du manchon intermédiaire 66. Ce filetage est formé par une rainure hélicoïdale ménagée dans l'épaisseur du couvercle. Cette rainure hélicoïdale a un pas égal au pas du filetage 90. Elle s'enroule sur 360° suivant la longueur filetée du couvercle.

Le couvercle arrière 64 comporte dans la partie médiane du tronçon tubulaire 100 une première paire de logements 107 diamétralement opposés adaptés pour recevoir les saillies 88 prévues aux extrémités des jambes 87 lorsque la seringue est dans son état initial.

En outre, une seconde paire de logements 108 diamétralement opposés est ménagée sur la surface cylindrique intérieure du couvercle 64 légèrement en avant du tronçon cylindrique 101. Ces logements 108 sont adaptés pour recevoir les saillies 88 et ainsi immobiliser axialement le manchon intermédiaire en fin de reconstitution du mélange extemporané.

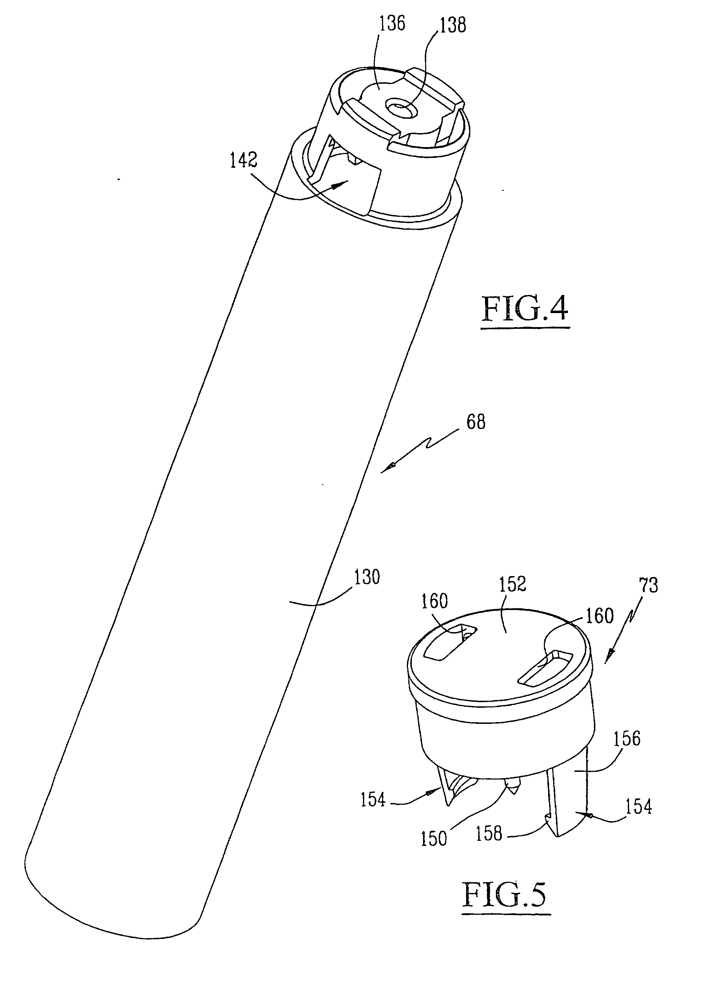

En outre, le couvercle arrière 64 présente en avant du fond 102 deux lumières 109 diamétralement opposées pour la réception des extrémités de crochets des moyens de verrouillage de l'organe de blocage 73. Cet organe 73 sera décrit dans la suite de la description, notamment en regard de la figure 5.

Les lumières 109 sont ménagées au travers du tronçon tubulaire 100 à la base du tronçon cylindrique 101.

En outre, dans le prolongement des lumières 109, le tronçon cylindrique 101 de diamètre réduit présente des méplats extérieurs 110 réduisant l'épaisseur de la paroi délimitant le tronçon cylindrique 101.

La distance séparant ces méplats opposés 110 est inférieure au diamètre extérieur du manchon intermédiaire 66.

Telle que représentée sur la figure 1, la cartouche 12 est reçue à l'intérieur du boîtier 14 et est maintenue entre, à l'avant, la paroi obturante 76 et, à l'arrière, les moyens d'actionnement 70 du piston arrière de la cartouche.

Ces moyens d'actionnement 70 comportent essentiellement un ressort d'actionnement 112 dans lequel est disposé un poussoir 114. Ce dernier comporte une tige 116 munie à son extrémité avant d'une pastille 117 d'appui sur la cloison arrière 30 de la cartouche.

La tige 116 est prolongée extérieurement par deux languettes élastiques 118 s'étendant sensiblement parallèlement. Ces languettes sont représentées à plus grande échelle sur la figure 1 B. Elles portent chacune à leur extrémité une lèvre extérieure 120 présentant en section une portion arrière biseautée 122 et un front avant 124 définissant un épaulement radial. Les languettes 118 sont à l'état initial engagées au travers du passage central 104 de telle sorte que les épaulements radiaux 124 s'appuient sur le fond 102 et ainsi maintiennent le ressort 112 bandé.

La poignée de préhension 68, représentée seule sur la figure 4, est formée essentiellement d'un manchon 130 entourant totalement le couvercle arrière 64.

La poignée 68 est obturée partiellement par une galette axiale 136 solidaire de l'extrémité arrière du manchon 130. La galette 136 est, à l'état initial, maintenue à une certaine distance du fond 102. La galette 136 comporte un conduit central 138 dans lequel sont partiellement engagées les extrémités biseautées des lèvres 120. Comme illustré sur la figure 1B, le conduit 138 s'évase progressivement vers son extrémité tournée vers les lèvres 120.

La poignée 68 comporte sur sa surface intérieure des canaux de guidage longitudinaux coopérant avec deux excroissances radiales 140 ménagées de part et d'autre du fond du couvercle arrière 64 afin d'assurer la solidarisation en rotation autour de leur axe de la poignée et du couvercle arrière.

La poignée de préhension 68 comporte sur sa surface latérale cylindrique, au voisinage de son extrémité arrière, deux lumières 142 diamétralement opposées pour le passage des jambes des moyens de verrouillage de l'organe de blocage 73.

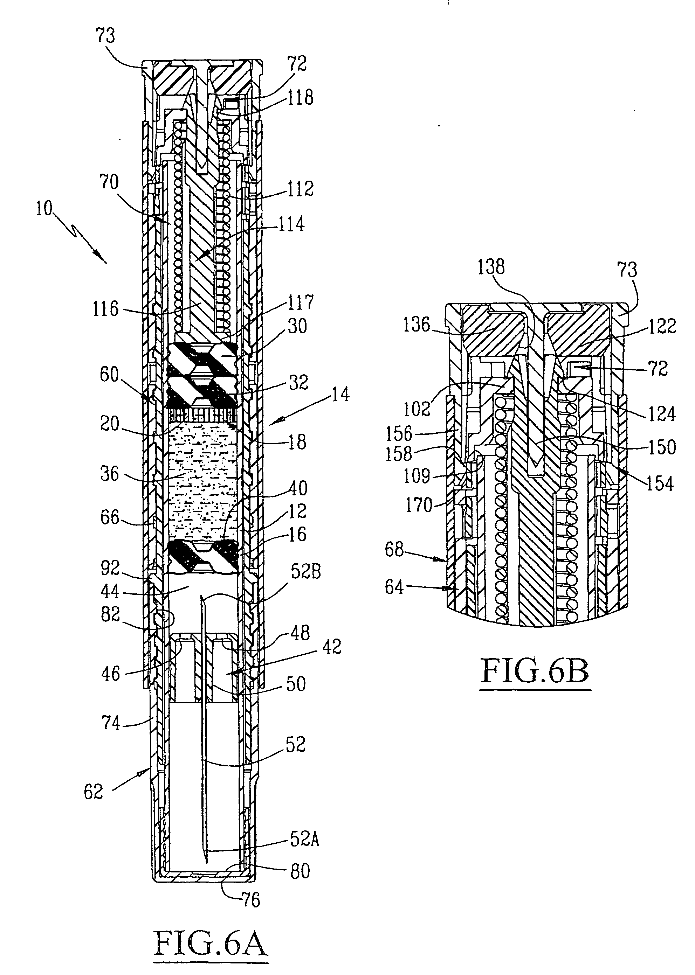

L'organe de blocage 73 est représenté seul sur la figure 5 et est visible avec le reste de la seringue sur les figures 1A, 1B, 6A et 6B.

L'organe de blocage comporte une broche axiale de sûreté 150 adaptée pour être engagée, à l'état initiale de la seringue, au travers du conduit central 138 de sorte que son extrémité s'étende entre les languettes 118 et maintiennent celles-ci écartées l'une de l'autre. La broche de sûreté 150 est venue de matière avec une coupelle 152 coiffant l'extrémité arrière de la poignée de préhension 68.

L'organe de blocage 73 présente en outre deux crochets 154 diamétralement opposés s'étendant depuis le bord de la coupelle 152 suivant des directions sensiblement parallèles à l'axe de la broche 150.

Ces crochets 154 comportent chacun une jambe 156 déformable élastiquement. Chaque jambe est munie à son extrémité libre d'une saillie 158 ménagée sur sa surface intérieure.

Au repos, les jambes 156 sont inclinées l'une vers l'autre en direction de leur extrémité libre portant les saillies 158.

Pour faciliter le démoulage des crochets, le fond de la coupelle 152 présente deux lumières 160 visibles sur la figure 5.

Comme illustré sur la figure 1, les saillies 158 des crochets sont adaptées pour être reçues, avant reconstitution, dans les lumières 109, afin d'assurer une immobilisation axiale de l'organe de blocage 73 par rapport au corps de la seringue et ainsi un verrouillage de l'organe de blocage, interdisant son retrait manuel alors que la reconstitution du mélange extemporané n'est pas achevée et en particulier lorsque la seringue est dans son état initial illustré sur la figure 1A.

Afin de procéder au montage de la seringue d'auto-injection représentée sur la figure 1A, on emmanche d'abord le couvercle arrière 64 dans la poignée de préhension 68. On introduit ensuite à l'intérieur du couvercle arrière 64 le ressort d'actionnement 112 et le poussoir 114, de telle sorte que les lèvres 120 des languettes 118 traversent le passage central 104 et que les épaulements radiaux 124 de celles-ci s'appliquent contre le fond 102 du couvercle arrière. L'organe de blocage 73 est alors mis en place à l'extrémité du couvercle arrière 65. La broche 150 s'étend entre les languettes 118, pour garantir leur écartement et ainsi interdire la libération accidentelle du ressort 112.

De plus, les saillies 158 ménagées aux extrémités des crochets pénètrent dans les lumières 109 sous l'effet de l'élasticité propre des jambes 156. Ainsi, les crochets se trouvent enclenchés élastiquement dans les lumières 109 à l'extrémité du couvercle arrière 64 assurant une retenue de l'organe de blocage à l'extrémité du couvercle arrière.

Le manchon intermédiaire 66 est ensuite vissé dans le couvercle arrière 64 par mise en prise des filetages complémentaires 90 et 106.

Le vissage s'effectue jusqu'à ce que les saillies 88 soient reçues dans le premier couple de logements 107 du couvercle arrière. Dans cette position, le tronçon fileté du couvercle arrière 64 s'étend dans la partie arrière du filetage 90 ménagé suivant la longueur du manchon intermédiaire.

La bague de sécurité 94 est ensuite mise en place entre la butée 92 et l'extrémité ouverte de la poignée 68.

Les moyens d'actionnement 70 étant immobilisés, la cartouche 12 complète est mise en place dans le manchon intermédiaire 66. L'extrémité avant de la cartouche est ensuite coiffée par le couvercle avant 62 muni de la membrane 80. Le couvercle 62 est alors vissé sur le manchon intermédiaire 66, assurant ainsi leur solidarisation définitive.

On comprend que dans cette configuration, la seringue d'auto-injection a tous ses éléments mobiles bloqués les uns par rapport aux autres et peut donc être transportée et mise en vente sans présenter aucun risque de déclenchement accidentel.

Lorsqu'un utilisateur souhaite s'injecter à lui-même la substance active contenue dans la cartouche d'injection 12, il retire la bague de sécurité 94.

Après retrait de la bague de sécurité 94, le manchon intermédiaire 66 peut être enfoncé à l'intérieur du couvercle arrière 64 par vissage. A cet effet, le couvercle avant 62 est actionné manuellement en rotation. II entraîne le manchon 66 auquel il est lié.

Lors du vissage, le tube 16, en appui sur le couvercle avant 62, est déplacé axialement par rapport au couvercle arrière 64.

La cloison 30 qui est maintenue en appui sur le poussoir 114, lui-même immobilisé par rapport au couvercle arrière 64, se trouve ainsi progressivement enfoncée suivant un trajet rectiligne à l'intérieur du tube 16, ce dernier étant déplacé vers l'arrière de la seringue.

Du fait de la présence du liquide 36, le piston intermédiaire 32 est également immobilisé par rapport au couvercle arrière 64.

L'enfoncement du tube 16 dans le couvercle arrière 64 conduit à un déplacement relatif entre le piston intermédiaire 32 et le tube 16.

Ainsi, le piston 32 se trouve amené dans le tronçon 18 du tube. Simultanément, la poudre 38 et la cloison avant 40 sont déplacées plus avant dans le tube. Une fois engagé dans le tronçon 18, le piston intermédiaire laisse libre les canaux définis entre les nervures 20.

La poursuite du vissage permet au fluide 36 contenu dans la chambre 34 de circuler vers la poudre 38 en passant dans les canaux 22 définis entre les nervures 20.

Progressivement, lors du vissage, la cloison arrière 30 se trouve enfoncée dans le tube suivant une course de préparation du mélange extemporané. Il chasse ainsi le liquide 36 au-delà du piston intermédiaire 32, ce dernier restant immobilisé dans le tronçon 18 du tube. L'arrivée du liquide entre le piston intermédiaire 32 et la paroi avant 40 provoque un déplacement de la paroi avant 40 vers l'avant du tube.

Au fur et à mesure de l'arrivée du liquide 36, la cloison mobile 40 est repoussée vers le porte-aiguille 42. L'air contenu dans l'espace 44 s'échappe progressivement par les évents 48 ménagés au travers du porte-aiguille 42. A cet effet, afin de faciliter l'écoulement de l'air depuis l'avant du tube vers l'extérieur, le couvercle 62 peut être muni de trous de très faible diamètre si les espaces entre les portions filetées coopérantes ne permettent pas un écoulement suffisant.

Pour assurer un transfert complet de la substance 36 dans la chambre contenant la poudre, le couvercle arrière 64 est vissé sur environ deux tours.

A la fin du vissage, comme illustré sur les figures 6A et 6B, la cloison arrière 30 atteint l'extrémité de sa course de préparation du mélange extemporané. Elle est alors en contact du piston intermédiaire 32 alors que la cloison avant 40 s'étend immédiatement en arrière de la pointe 52B de l'aiguille. Dans cette position, la quasi-totalité du liquide 36 retenu initialement dans la chambre 34 est transférée entre le piston intermédiaire 32 et la cloison avant 40.

Dans la phase finale de l'enfoncement du manchon intermédiaire 66 dans le couvercle arrière 64, l'extrémité arrière du manchon entre en contact avec les saillies 158 des crochets engagés dans les lumières 109. Sous l'action du déplacement du manchon intermédiaire, les saillies sont progressivement repoussées vers l'extérieur totalement en dehors des lumières 109.

A l'issue du vissage, l'extrémité arrière du manchon intermédiaire 66 vient s'appuyer sur le couvercle arrière et en particulier contre l'épaisseur du tronçon cylindrique 101. Dans cette position, les plages notées 170, formant des surfaces de dégagement, délimitées à la surface extérieure du manchon intermédiaire 66, entre son extrémité arrière et le bord de la lumière 86 s'étendent dans les lumières 109 aux emplacements initialement occupés par les saillies 158 des crochets.

La présence de ces surfaces 170 dans les lumières 109 assure un maintien des saillies 158 à l'écart du prolongement de la surface latérale du couvercle arrière 64, libérant ainsi l'enclenchement élastique des crochets 154 dans les lumières 109.

On conçoit que, dans cette position, l'organe de blocage 73 peut être retiré manuellement par traction sur celui-ci et extraction suivant l'axe de la seringue.

Lors du retrait de l'organe de blocage, la broche de sûreté se trouve retirée de l'espace entre les languettes 118, libérant ainsi celles-ci.

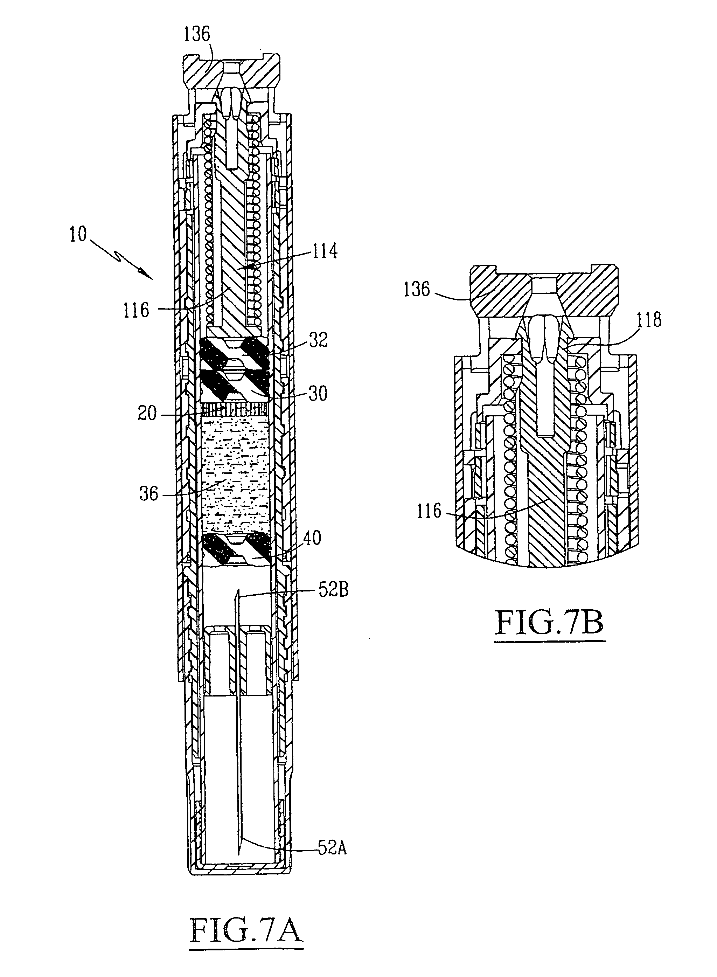

Afin de procéder à l'injection proprement dite, l'extrémité avant de la seringue telle qu'illustrée sur les figures 7A et 7B est appuyée contre la zone du corps où doit être effectuée l'injection. L'opérateur presse alors la poignée de préhension 68 vers l'avant. Ainsi, la galette 136 agit sur les languettes élastiques 118 et conduit au rapprochement des lèvres 120. Lorsque celles-ci sont suffisamment rapprochées, le poussoir 114 est libéré du passage 104 sous l'effet du ressort d'actionnement 122 comprimé. Ce ressort repousse alors le poussoir 114. Ce dernier agit sur les éléments contenus dans le tube 16 et provoque le déplacement de ceux-ci vers l'aiguille 52. En particulier, la cloison avant 40 atteignant la pointe 52B de l'aiguille, s'empale sur celle-ci, provoquant ainsi la mise en contact de l'aiguille avec la substance active reconstituée formée du mélange de la poudre 38 et du liquide 36. Le porte-aiguille 42 est ensuite projeté vers l'extrémité avant du tube 16. L'aiguille 52 traverse alors la membrane 80 puis la paroi obturante 76 et pénètre enfin dans les tissus de l'utilisateur.

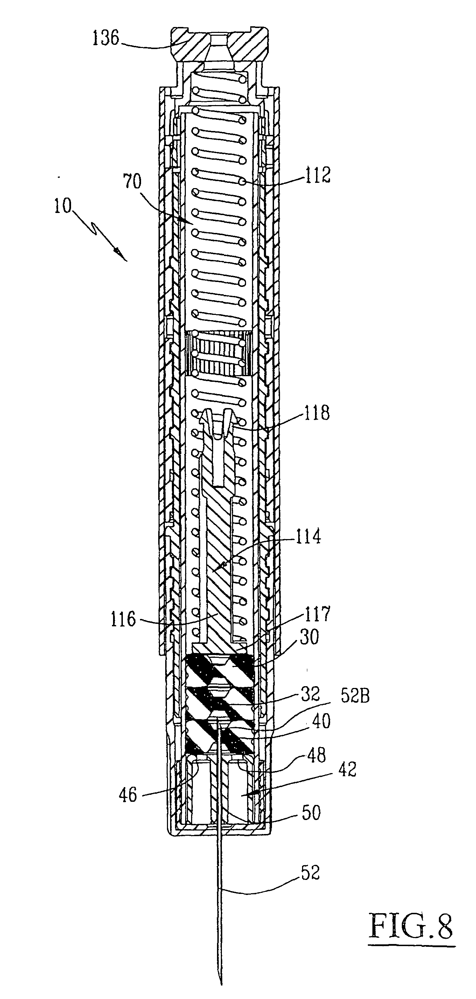

La suite de la détente progressive du ressort d'actionnement 112 conduit la substance active contenue entre le piston intermédiaire 32 et la cloison avant 40 à être injectée dans les tissus de l'utilisateur au travers de l'aiguille d'injection 52. En fin d'injection, la seringue est dans la position représentée sur la figure 8. L'étape d'injection proprement dite s'effectue de manière très rapide dès que le ressort est libéré, supprimant toute sensation de douleur pour l'utilisateur.

On comprend qu'avec une seringue telle que décrite ici, l'organe de blocage 73 étant équipé de moyens de verrouillage interdisant son retrait tant que la reconstitution du mélange extemporané n'est pas achevé, le déclenchement des moyens automatiques d'injection du mélange extemporané ne peut avoir lieu avant que la reconstitution ne soit achevée, puisque l'organe de blocage interdit ce déclenchement et que cet organe de blocage ne peut pas être retiré avant l'achèvement de la reconstitution.

Ainsi, si l'opérateur cherchait à procéder à l'injection alors que la reconstitution n'est pas achevée, celui-ci ne pourrait retirer l'organe de blocage et ne pourrait donc déclencher les moyens d'injection automatiques et, de ce fait, risquer de s'injecter un produit non reconstitué. Ne pouvant retirer l'organe de blocage, l'opérateur comprendra que la reconstitution du mélange extemporané n'est pas achevée et il poursuivra la rotation des deux parties complémentaires du corps afin d'effectuer complètement la reconstitution, après quoi seulement il pourra retirer l'organe de blocage et ensuite provoquer l'injection automatique du mélange extemporané.

## Revendications

1. Seringue d'auto-injection (10) d'un mélange extemporané, du type comportant :
- un corps (60) renfermant au moins deux substances (36, 38) initialement séparées par un piston intermédiaire (32), lequel corps (60) comporte deux parties mobiles (64, 66) l'une par rapport à l'autre, entre une position initiale dans laquelle les deux substances (36, 38) sont séparées et une position finale dans laquelle les deux substances (36, 38) sont mélangées pour former un mélange extemporané ;
- des moyens automatiques (70, 72) d'injection du mélange extemporané hors du corps (60) afin de procéder à l'injection ; et
- des moyens (73) de blocage des moyens automatiques d'injection (70, 72) interdisant leur déclenchement, les moyens de blocage comportant un organe de blocage (73) déplaçable entre une position active de blocage des moyens automatiques d'injection (70, 72), et une position inactive de blocage des moyens automatiques d'injection (70, 72)
**caractérisée en ce qu'**elle comporte des moyens (154, 109) de verrouillage des moyens de blocage (73) interdisant le déblocage des moyens automatiques d'injection (70, 72) avant la fin du mélange des deux substances, les moyens de verrouillage comportant des profils complémentaires d'accrochage par enclenchement élastique prévus sur ledit organe de blocage (73) et sur une première partie mobile (64) du corps, lesquels profils complémentaires d'accrochage (109, 158) sont normalement en prise, et **en ce que** la seconde partie mobile (66) du corps comporte au moins une surface (170) de dégagement des profils complémentaires d'accrochage adaptés pour assurer le dégagement desdits profils complémentaires d'accrochage (109, 158), seulement lorsque les deux parties mobiles (64, 66) du corps sont dans leur position finale.

2. Seringue d'auto-injection selon la revendication 1, **caractérisée en ce que** lesdits profils complémentaires d'accrochage comportent, d'une part, au moins un crochet (154) solidaire dudit organe de blocage (73), et, d'autre part, au moins une lumière (109) ménagée dans ladite première partie mobile (64), le ou chaque crochet (154) étant sollicité élastiquement pour être reçu au moins partiellement dans une lumière associée (109), et **en ce que** la ou chaque surface de dégagement (170) est adaptée pour s'étendre dans la ou chaque lumière (109) et repousser totalement le ou chaque crochet associé hors de la lumière (109), seulement lorsque les deux parties mobiles (64, 66) sont dans leur position finale.

3. Seringue d'auto-injection selon la revendication 1 ou 2, **caractérisée en ce que** ledit organe de blocage (73) est amovible.

## Patentansprüche

1. Autoinjektionsspritze (10) zur automatischen Injektion einer Sofortanfertigungs- und Verabreichungsmischung, einer Art die aufweist:
- einen Körper (60), der wenigstens zwei Substanzen (36, 38) einschließt, die zu Anfang durch einen Zwischenkolben (32) getrennt sind, wobei der Körper (60) zwei bewegliche Teile (64, 66) aufweist, die in Bezug aufeinander zwischen einer Ausgangsstellung, in der die beiden Substanzen (36, 38) getrennt sind, und einer Endstellung, in der die beiden Substanzen (36, 38) zur Bildung einer Sofortanfertigvings- und Verabreichungsmischung gemischt sind, beweglich sind;
- Automatische Injektionsmittel (70, 72) zur automatischen Injektion der außerhalb des Körpers (60) angefertigten Sofortanfertigungs- und Verabreichungsmischung, um die Injektion vorzunehmen, und
- Mittel (73) zum Blockieren der automatischen Injektionsmittel (70, 72), die deren Auslösen verbieten, wobei die Blockiermittel eine Blockiereinrichtung (73) aufweisen, die zwischen einer aktiven Stellung hinsichtlich der Blockierung der automatischen Injektionsmittel (70, 72), und einer inaktiven Stellung hinsichtlich der Blockierung der automatischen Injektionsmittel (70, 72) verschiebbar sind,
**dadurch gekennzeichnet, dass** sie Mittel (154, 109) zur Verriegelung der Blockiermittel (73), welche die Deblockierung der automatischen Injektionsmittel (70, 72) vor dem Ende des Mischens der beiden Substanzen verbieten, aufweist, wobei die Verriegelungsmittel komplementäre Profile zur Verhakung durch elastische Verrastung, vorgesehen an der Blockiereinrichtung (73) und einem ersten beweglichen Teil (64) des Körpers, aufweisen, wobei die komplementären Verhakungsprofile (109, 158) im Normalzustand im Eingriff sind, und dass das zweite bewegliche Teil (66) des Körpers wenigstens eine Fläche (170) zum Lösen der komplementären Verhakungsprofile aufweist, die zu Gewährleistung des Lösens der komplementären Verhakungsprofile (109, 158) nur, wenn die beiden beweglichen Teile (64, 66) des Körpers sich in ihrer Endstellung befinden, eingerichtet sind.

2. Autoinjektionsspritze nach Anspruch 1, **dadurch gekennzeichnet, dass** die komplementären Verhakungsprofile einerseits wenigstens einen mit der Blockiereinrichtung (73) fest verbundenen Haken (154) und andererseits wenigstens ein in dem ersten beweglichen Teil (64) ausgebildetes Loch (109) aufweisen, wobei der oder jeder Haken (154) elastisch so belastet ist, dass er wenigstens teilweise in dem zugeordneten Loch (109) aufgenommen ist, und dass die oder jede, Lösefläche (170) so eingerichtet ist, dass sie sich in das oder jedes Loch (109) erstreckt und dabei den oder jeden zugeordneten Haken nur dann aus dem Loch (109) drückt, wenn sich die beiden beweglichen Teile (64, 66) in ihrer Endstellung befinden.

3. Autoinjektionsspritze nach Anspruch 1 oder 2, **dadurch gekennzeichnet, dass** die Blockiereinrichtung (73) abnehmbar ist.

## Claims

1. Syringe for the automatic injection (10) of an extemporaneous mixture, of the type comprising :
- a body (60) containing at least two substances (36, 38) initially separated by an intermediate piston (32), the said body comprising two parts (64, 66) which are movable relative to each other between an initial position in which the two substances (36, 38) are separated and a final position in which the two substances (36, 38) are mixed to form an extemporaneous mixture:
- means (70, 72) for automatically injecting the extemporaneous mixture outside the body (60) so as to inject; and
- means (73) for blocking the automatic injection means (70, 72) preventing their release, comprising a blocking device (73) which is movable between an active position for blocking the automatic injection means (70, 72) and an inactive position for blocking the automatic injection means (70, 72),
**characterised in that** it comprises means (154, 109) for locking the blocking means (73) preventing the release of the automatic injection means before the two substances have been fully mixed, the locking means comprising complementary profiles for hooking by elastic engagement provided on the said blocking device (73) and on a first movable part (64) of the body, the said complementary hooking means (109, 158) being normally engaged, and **in that** the second movable part (66) of the body comprises at least one surface (170) for releasing the complementary hooking means adapted to ensure the release of the said complementary hooking means (109, 158) only when the two movable parts (64, 66) of the body are in their final position.

2. Auto-injector syringe according to claim 1, **characterised in that** the said complementary hooking means comprise, firstly, at least one hook (154) integral with the said blocking device (73) and, secondly, at least one aperture (109) arranged in the said first movable part (64), the or each hook (154) being elastically urged to be at least partially received in an associated aperture (109), and **in that** the or each releasing surface (170) is adapted to extend into the or each aperture (109) and to totally push the or each associated hook outside the aperture (109), only when the two movable parts (64, 66) are in their final position.

3. Auto-injector syringe according to claim 1 or 2, **characterised in that** the said blocking device (73) is detachable.
